# EUROPEAN PATENT APPLICATION

(11) **EP 4 552 653 A1**
(43) Date of publication of application: **14.05.2025**
(21) Application number: 24211570.7
(22) Date of filing: 07.11.2024
(51) Int. Cl.: A61K 47/61, A61K 9/00, A61K 31/728, A61K 47/36

(54) **HYALURONIC ACID DERIVATIVES PHOTOGENERATING NITRIC OXIDE**

(30) Priority: 07.11.2023 IT 202300023433
(71) Applicant: Università degli Studi di Catania, 95131 Catania (IT)
(72) Inventor: SORTINO, Salvatore, 95121 Acicastello (CT) (IT); QUAGLIA, Fabiana, 80127 Napoli (NA) (IT)
(74) Representative: Primiceri, Maria Vittoria

(57) **Abstract**

The present invention describes conjugates between hyaluronic acid and nitric oxide (NO) photodonor compounds of general formula (I): 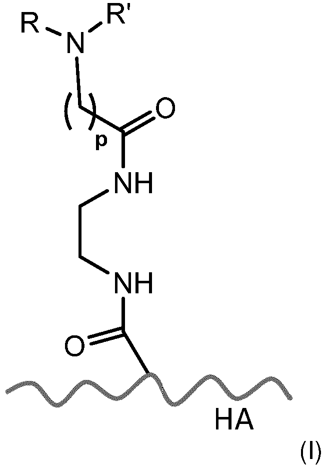 wherein:
HA = hyaluronic acid backbone
p = positive integer between 3 and 16
R = H, NO
R' = nitrophenyl substituted with, in ortho position with respect to the nitro group, one or two methyl or trifluoromethyl groups, preferably 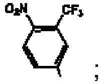 nitrobenzofurazane,

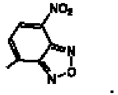

The conjugates are activated by visible light, are effective for the repair and regeneration of skin tissue and have a variety of uses in the pharmaceutical and cosmetic fields.

The invention also relates to cosmetic and pharmaceutical compositions, preferably for topical application, to be used for all purposes that benefit from the release of NO at the skin level in order to preserve the integrity and functionality of the skin.

## Description

### Technical field

The present invention relates to the production of hyaluronic acid derivatives. In particular, the invention relates to hyaluronic acid derivatives (hereinafter also (HA)) containing groups capable of releasing nitric oxide (NO) under ambient light conditions. More specifically, the invention refers to conjugates between low molecular weight hyaluronic acid and NO donors activated by ambient light for the repair and regeneration of skin tissue.

### Prior art

Hyaluronic acid (HA) is a linear polysaccharide of N-acetyl glucosamine and glucuronic acid in alternating sequence. HA is an important mediator of physiological and pathological processes. An alteration in the synthesis or degradation of this polymer can cause disorders and pathological events [1,2]. Within the organism, HA is responsible for different biological actions that depend mainly on its molecular weight (MW). In general, HA can behave as a structural molecule or can be involved in cellular signaling thanks to its ability to interact with specific membrane receptors, among which the main one is the CD44 receptor (cluster of differentiation 44).

Müller-Lierheim (Why chain length of hyaluronan in eye drops matters Diagnostics, 10 (8) (2020*)* classify HA according to MW into 5 categories. Very high MW (vHMM-HA >5000 kDa) and High MW HA (HMW-HA, 1000-3000 kDa) with cytoprotective and anti-inflammatory activity, Medium MW HA (MMW-HA, 250-1000 kDa) with pro-inflammatory effects, Low MW HA (LMW-HA, 10-250 kDa) with immunostimulatory properties and able to promote nitric oxide production and keratinocyte migration/proliferation, as well as promote angiogenesis, and Oligo-HA (MW < 10 kDa) able to promote wound healing.

The amount of HA in human skin represents one third of the entire content present in the organism *https:*//*doi.org*/*10.1111*/*srt.12228.* Cells do not always produce HA efficiently throughout life, and decreased HA levels result in myofascial stiffness, skin aging, dryness, and wrinkle formation *https:*//*doi.org*/*10.1111*/*srt.12228.*

Intermediate MW HA (MMW-HA) and low MW HA (LMW-HA) are used in cosmetic creams that promote skin hydration, skin elasticity regeneration and to combat aging *(*Papakonstantinou, E.; Roth, M.; Karakiulakis, G. Hyaluronic acid: A key molecule in skin aging. Derm. Endocrinol. 2012, 4, 253-258*).*

At the skin level, HA has a capacity to penetrate intact skin strictly determined by its MW as shown by *Essendoubi* *https:*//*doi.org*/*10.1111*/*srt.12228.*

HMW-HA, due to its high molecular size, is unable to penetrate the deeper layers of the skin, remaining localized at the superficial level where it forms a viscoelastic film and mainly mediates a hydrating action.

Intermediate molecular weight HA penetrates deeper into the skin and continues to act by retaining the water contained in the skin through its hygroscopicity, in addition to being involved in the cellular repair process.

LMW-HA is instead able to penetrate deeper into the skin and also to interpose itself between the keratinocytes, fortifying the tight-junctions so as to intensify the strength of the skin barrier and further prevent the removal of water, in addition to stimulating the production of collagen *(*Papakonstantinou, E.; Roth, M.; Karakiulakis, G. Hyaluronic acid: A key molecule in skin aging. Derm. Endocrinol. 2012, 4, 253-258*)* and reduce the damage caused by aging and solar radiation [3]. LMW-HA, as shown by recent studies, is also able to influence the expression of genes that encode proteins involved in the cellular differentiation that keratinocytes undergo during their migration towards the stratum corneum (SC) [3].

HA intervenes in different phases of the wound repair process (ACS Appl. Bio Mater. 2021, 4, 1, 311-324*).* In the hemostasis phase, HMW-HA promotes coagulation and is then catabolized to fragments < 120 kDa with immunoregulatory and proangiogenic action and, subsequently, to oligomers (6-20 monomers) that initiate cell proliferation, promoting collagen deposition and tissue remodeling.

Several hyaluronic acid-based products for the treatment of acute and chronic wounds are commercially available (e.g. Hyalosafe^{®}, Hyalomatrix^{®}, HylaSponge^{®}, Connettivina^{®})

Therefore, HA is a polymer with numerous properties ranging from the ability to maintain a high level of skin hydration and reduce the signs of aging, to that of stimulating cell growth promoting tissue regeneration, improving adhesion between cells and contributing to the functioning of various membrane receptors.

HA used in the present invention may derive from any source; for example, it may be produced by extraction from rooster combs (EP 138572 B1), fermentation (EP 716688 B1) or biotechnology, and have a molecular weight between 400 and 3×10⁶ Da, preferably between 200,000 and 750,000 Da, and even more preferably between 8000 Da and 15,000 Da.

The derivatization reaction according to the invention may be applied both to the polysaccharide as is and to the previously modified polysaccharide. From HA, molecular networks will then be obtained that are variously modified according to known methods, in particular:
HA salified with organic and/or inorganic bases (EP 138572 B1);
HYAFF^{®}: HA esters with alcohols of the aliphatic, arylaliphatic, cycloaliphatic, aromatic, cyclic and heterocyclic series, with an esterification percentage that can vary, depending on the type and length of the alcohol used, preferably between 50 and 100%, while the remaining percentage of non-esterified HA can be salified with organic and/or inorganic bases (EP 216453 B1);
HYADD^{®}: HA amides with amines of the aliphatic, arylaliphatic, cycloaliphatic, aromatic, cyclic and heterocyclic series, with an amidation percentage between 0.1 and 50%, while the remaining percentage of non-amidated HA can be salified with organic and/or inorganic bases (EP 1095064 B1);
O-sulfated derivatives of HA up to the 4th degree of sulfation (EP 702699BI);
ACP^{®}: internal esters of HA with an esterification percentage not exceeding 20%, preferably between 0.05 and 10% of esterification, while the remaining percentage of non-esterified HA can be salified with organic and/or inorganic bases (EP 341745 B1);
Deacetylated HA: they derive from the deacetylation of N-acetylglucosamine residues present in HA, with a deacetylation percentage preferably between 0.1 and 30%, while all the carboxylic groups of HA can be salified with organic and/or inorganic bases (EP 1313772 B1);
HYOXX: percarboxylated derivatives of HA obtained by oxidation of the primary hydroxyl of the N-acetylglucosamine fraction with a degree of percarboxylation between 0.1 and 100%, and preferably between 25 and 75%. All carboxylic groups of HA are salificable with organic and/or inorganic bases (EP 1339753 A).

As already mentioned, the derivatives according to the invention, obtained after derivatization of hyaluronic acid, retain the biological properties of the starting polysaccharide. It is therefore possible to select the most suitable derivative, depending on the type of application chosen for the final product.

Nitric oxide (NO) is a gaseous free radical that plays a key role in the regulation of a variety of biological processes including vasodilation, neurotransmission and hormone secretion [4]. It is a ubiquitous compound produced by numerous cells in the body that contain enzymes of the NO synthase (NOS) family. Although it is produced ubiquitously, one of the tissues wherein NO production occurs most intensely is the skin wherein most of the cells present (keratinocytes, Langerhans cells, melanocytes, fibroblasts, neutrophils, macrophages) contain NOS enzymes.

At the skin level, NO plays an important role in preserving the integrity and functionality of the skin. It is therefore evident that a dysregulation in its production and in the signaling mediated by it is associated with the development of possible pathological conditions [5]. Although it is a gaseous molecule with a short half-life (about 5 seconds) [4], the skin has the ability to accumulate NO in the form of S-nitroso-glutathione.

A product believed to be able to generate NO at the skin level is described in document WO2020227367A. This invention allows to generate NO only on the surface of the skin through an instantaneous chemical reaction between nitrites and appropriate reducing agents such as ascorbic acid and metabisulphite. The proposed system does not contain HA, allows to generate NO only on the skin surface and exclusively in an instantaneous manner, not allowing to regenerate the epidermis in depth and to act in a prolonged manner.

To date, the only product on the market capable of generating NO at the skin level is N1O1 (Pneuma Nitric Oxide, LLC, Austin Texas)-Nitrix Oxide Activating Serum for cosmetic use. The product does not contain HA and is made up of different components, contained in two different compartments, which once mixed on the skin allow NO to be generated only on the surface of the skin through an instantaneous chemical reaction.

WO2009003624 describes derivatives of hyaluronic acid functionalized with S-nitroso-thiol groups for dermatological and cosmetic use. In particular, WO2009003624 discloses the use of HA derivatives, administered in the form of injectable gel, hydrogel, creams, medications or films for topical application, for the treatment of skin lesions or defects, tissue biorevitalization or tissue healing. The release of NO from the derivatives is spontaneous (for a derivative the release process is complete in 500 minutes) and therefore uncontrollable. This peculiar property determines a serious problem of stability over time of formulations containing such derivatives, in addition to the fact that it makes it extremely complex to plan a dosage regimen. The same patent document also reports studies on the amount of NO spontaneously released by hyaluronic acid derivatives through indirect spectrophotometric detection analysis (540 nm) by Griess assay.

WO2019121488A1 describes hyaluronic acid esters functionalized with molecules that release NO (S-nitroso-thiol groups as in document WO'624) and are used in ophthalmology. High molecular weight HA (HMW-HA) is used. The HMW-HA derivatives object of the invention described therein, present the same limit as the derivatives in WO2009003624 of spontaneously releasing NO in an uncontrolled manner.

Organic NO-donating compounds are known [6-11]. It should be noted, however, that the conjugation of a photoactive molecule with a polymeric skeleton does not necessarily determine a retention of the photochemical properties of the photoactive unit since the polymeric network can significantly interfere with the efficiency and nature of the photoinduced processes (V. Ramamurthy, Photochemistry in Organized and Constrained Media 1st Edition).

There is a need to overcome the drawbacks of prior art.

It has now been found that some derivatives obtained by conjugation between HA and nitric oxide photodonors release NO when activated by ambient light and are potentially effective for skin tissue repair and regeneration. Such conjugates have a variety of uses in the pharmaceutical, cosmetic and medical fields.

Controlling the release by an external stimulus such as ambient light directly into the skin allows to benefit from the combined action of HA and the release of NO directly at the site of action. The use of a biocompatible visible light source after skin application of the derivative can enhance the release of NO and amplify its beneficial effects. The activation of NO release by light radiation also allows to increase the stability of spontaneous conjugates during storage in the presence of solvents typically used in dermatological preparations.

It is possible to develop HA-photodonor conjugates from HA at different MW and, possibly, cross-linked to benefit from the combined biological action of HA and NO under ambient light, as well as determine the release of NO in specific areas reached by the conjugates. The HMW-HA derivatives of the invention, although they are unable to penetrate deeply through the skin, ensure the release of NO, avoiding spontaneous release during the preservation of the skin formulation. The mixture of HA derivatives with different MWs then allows the production of NO in superficial and deep areas of the skin, ensuring a broad biological effect.

To deliver HA-photodonor conjugates into the deep skin, a nanometric formulation has been prepared that also allows the transport to that level of a lipophilic molecule with synergistic activity and, potentially, hydrophilic molecules in combination.

Unless specifically excluded in the detailed description that follows, what is described in this chapter is to be considered as an integral part of the detailed description.

### Summary of the invention

The object of the present invention is therefore the creation of conjugates between hyaluronic acid and photodonor compounds of nitric oxide (NO) with electromagnetic radiation in the range between 300 and 800 nm, preferably in the range 400-700 nm. In particular, the conjugates of the invention are capable of releasing NO when stimulated by electromagnetic radiation in the range between 300 and 800 nm, preferably in the range 400-700 nm.

Such conjugates activated by ambient light are effective for the repair and regeneration of skin tissue and have a variety of uses in the pharmaceutical, cosmetic and medical fields.

Another object of the present invention are cosmetic and pharmaceutical compositions, preferably for topical application to be used for all purposes that benefit from the release of NO at the skin level in order to preserve the integrity and functionality of the skin. The compositions include the conjugates described by the formula (I) explained below in a cosmetically and pharmaceutically acceptable vehicle.

Yet another object of the present invention is the topical application of the compositions of the invention and their use in the animal and human fields, in particular for mammals, including humans, children, newborns and the elderly.

These and other objects, advantages and features of the present invention will be more fully described in a detailed description of the embodiments which follow, furthermore the claims describe preferred variations of the invention, forming an integral part of this specification.

### Brief description of the Figures

Further purposes and advantages of the present invention will be clear from the detailed description that follows of the examples of its embodiment (and its variants) and from the attached drawings, given purely for explanatory and non-limiting purposes, wherein:
**Figure 1****.** Molecular structures of HA derivatives 1 and 2.
**Figure 2****.** UV-Vis absorption spectra of derivatives 1 and 2 in water:methanol solution (1:1 v:v) and their overlap with the component of solar radiation capable of reaching the epidermis.
**Figure 3****.** Absorption spectra of conjugates 1 (A) and 2 (B) in water:methanol solution (1:1 v:v) observed at different irradiation times with visible light at 405 nm. The arrows indicate the progression of the spectral profile following the irradiation time.
**Figure 4****.** Photostimulated NO release from solutions of conjugates 1 (A) and 2 (B) monitored indirectly by Griess assay (A) and directly by amperometric sensor (B). Visible light excitation at 405 nm.
**Figure 5****.** Scheme of the operating principle of NO photorelease with fluorescent reporter in the case of conjugate 2.
**Figure 6****.** (A) Increase in fluorescence intensity of conjugate 2 in water:methanol solution (1:1 v:v) following irradiation with visible light (λ_{exc} = 430 nm). (B) Real images of the sample in (A) acquired under visible light illumination.
**Figure 7****.** Photochemical properties of the microemulsion containing conjugate 2. (A) Absorption spectra observed at different irradiation times with visible light at 405 nm. (B) Changes in absorbance at 380 nm under aerobic and anaerobic conditions following irradiation with visible light. (C) NO release profile observed following alternating irradiation with visible light. (D) Increase in emission intensity observed at different irradiation times with visible light.
**Figure 8****.** Absorption spectrum of conjugate 2 in the microemulsion in the dark and at room temperature over time.
**Figure 9****.** (A) Scheme of the application of the microemulsion on the skin and its light activation. (B) Permeation of the microemulsion with conjugate 2 on a porcine ear. The results are reported as µg/cm² of conjugate 2 permeated ± the standard deviation of three different experiments.
**Figure 10****.** Visualization of the fluorescent reporter in a porcine ear skin section after application of the microemulsion for 4 hours in the dark. The section was observed immediately (A) and after 90 seconds (B) and 180 seconds (C) of irradiation with blue light (λ_{exc} = 488 nm; 2 µsec pulses).
**Figure 11****.** Visualization of the fluorescent reporter in porcine ear skin after application of the microemulsion for 24 hours under ambient light. (A) Skin section; (B) concentration gradient of conjugate 2 in porcine ear skin (z-stack).

### Definitions

In the context of the present invention, the terms "derivative(s)" and "conjugate(s)" are to be considered synonymous.

In the context of the present invention, the term "hyaluronic acid" or "HA" is intended to indicate any form of hyaluronic acid such as, for example, those indicated in the chapter of the prior art, such as derivatives and salts of HA in all molecular weights, unless these are explicitly excluded.

In the context of the present invention, the term "donor HA conjugate(s)" is intended to indicate derivative(s) or conjugate(s) between hyaluronic acid and nitric oxide photodonor compounds wherein the bond or conjugation is of the covalent type.

In the context of the present invention, the term "deep skin" means the layer below the stratum corneum.

In the context of the present invention, the term "about" indicates a numerical value equal to the reported value ± its standard deviation.

### Detailed description

The invention consists in the production of conjugates between hyaluronic acid and nitric oxide donors activated by ambient light for the repair and regeneration of skin tissue. Said conjugates have the following general formula (I) 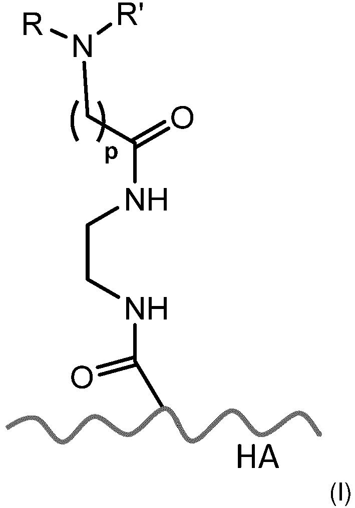 wherein:
HA = hyaluronic acid backbone
p = positive integer between 3 and 16
R = H, NO
R' = nitrophenyl substituted with, in ortho position with respect to the nitrogroup, one or two methyl or trifluoromethyl groups, preferably 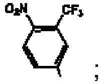 nitrobenzofurazane,

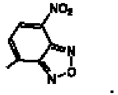

The preparation of the adducts of the invention is reported in the EXAMPLES section.

The molecular weight of HA may preferably range from 400 to 3×10⁶ Da, preferably from 200,000 to 750,000 Da, and even more preferably from 8000 Da to 15,000 Da, but also molecular weights outside (higher or lower) of these ranges may be suitable to be functionalized with the NO photodonor compounds according to the present invention.

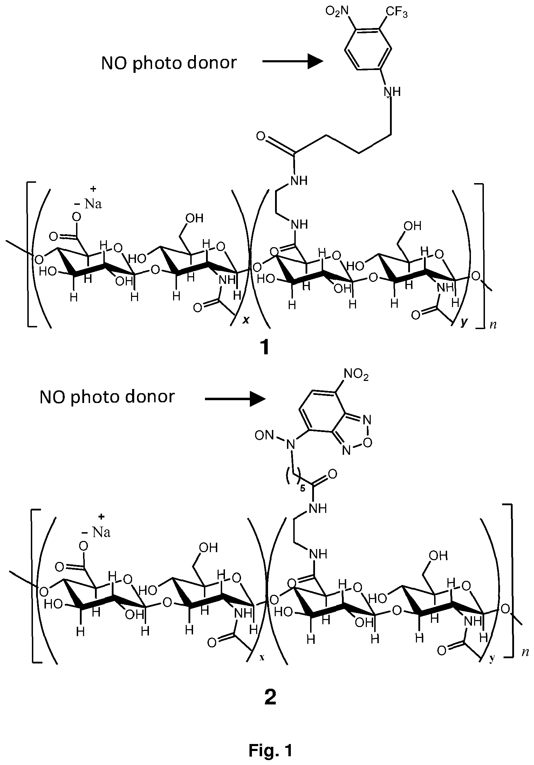

The preferred conjugates of the invention are illustrated by formulas 1 and 2 in Figure 1 and were obtained by functionalization of HA with NO photodonor compounds, as illustrated in the Examples section.

Conjugates 1 and 2 integrate in their structure a nitroaniline derivative and an N-nitrosamine benzofurazan as suitable NO photodonors.

All the described photodonors are activated by light in the visible spectrum.

It has been experimentally verified that the integration of photoactive units into the polymeric skeleton of HA through a simple synthetic protocol does not modify its light absorption properties. In the sense that it was already known from the literature that they released NO in the visible range. However, it was neither known nor deducible from the known art that the simultaneous delivery of NO and HA into the deep skin could occur and that the photoactivated release of NO occurred at the level of the dermis in a controlled manner. It should also be noted that the conjugation of a photoactive molecule with a polymeric skeleton does not necessarily determine a retention of the photochemical properties of the photoactive unit since the polymeric network can significantly interfere with the efficiency and nature of the photoinduced processes (V. Ramamurthy, Photochemistry in Organized and Constrained Media 1st Edition)

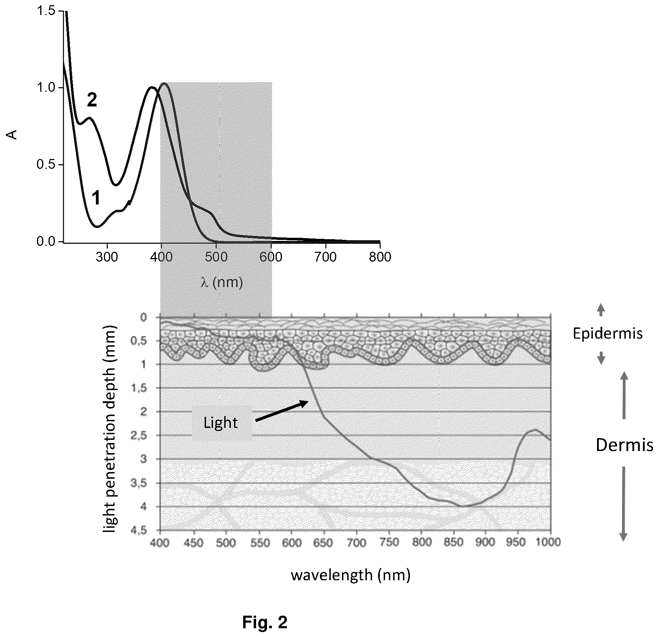

Figure 2 shows the UV-Vis absorption spectra of the two conjugates that show the characteristic bands typical of the individual NO photodonors, and their excellent overlap with the solar radiation component capable of reaching the deep skin.

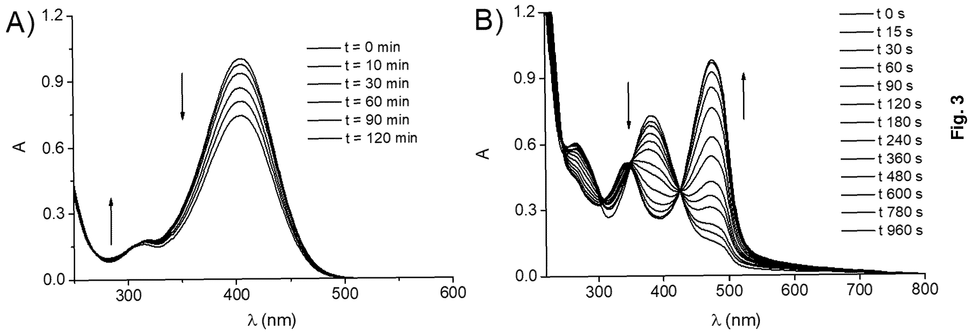

The irradiation of water:methanol solutions of conjugates 1 and 2 induces substantial spectral changes (Figure 3) with photolysis profiles characterized by clear isosbestic points and identical to those observed for the isolated photodonors [6-11]. This trend demonstrates that the photochemical and photophysical properties of the photodonors are preserved in the respective conjugates.

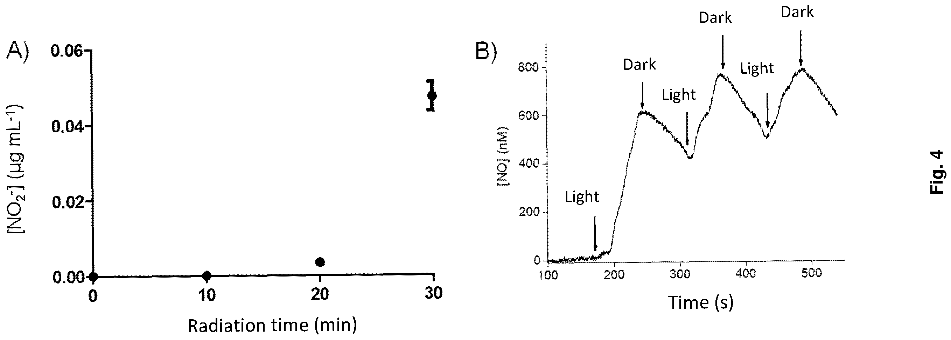

This result is consistent with processes involving the photorelease of NO from the nitro group of conjugate 1 and from the nitroso group of conjugate 2. Confirmation of NO generation following irradiation is provided by monitoring this species both indirectly and directly, using the Griess assay (Griess, P. (1879) Chem. Ber. 12, 426-8), which determines the nitrites formed following the reaction of the produced NO with oxygen, and amperometric detection using a NO-specific electrode (Methods Enzymol 2008;436:63-95), respectively. Figure 4 shows, as a non-limiting example, representative results obtained with the indirect method on conjugate 1 and with the direct method on conjugate 2. It is evident that NO release from both conjugates occurs exclusively under illuminated conditions and stops instantly in the dark. This result is not trivial since the photochemical properties of isolated molecules (the NO photodonors in the present case) can be significantly modified following their functionalization with other species (HA in the present case), as previously explained.

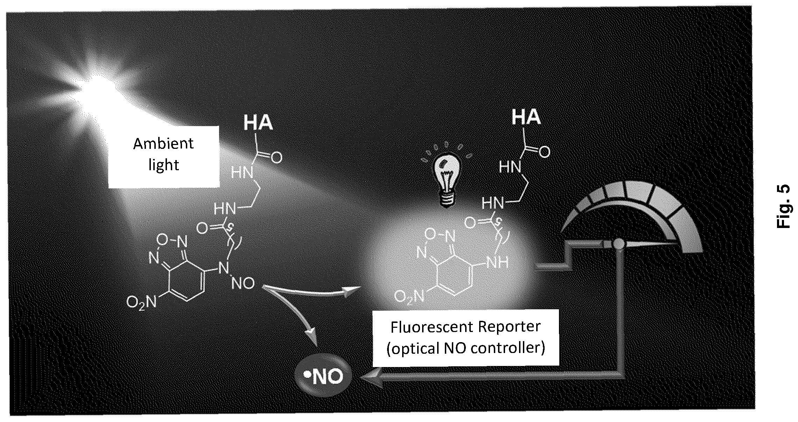

In order to be able to verify the release of NO in the different skin layers by confocal fluorescence microscopy, derivative 2 was equipped with a fluorescent reporting function [10,11]. Briefly, derivative 2 is not fluorescent but the product formed after the release of NO is highly fluorescent, as illustrated in Figure 5. This property allows to directly correlate the fluorescence signal with the site of NO generation and, in principle, to also monitor the amount of NO released.

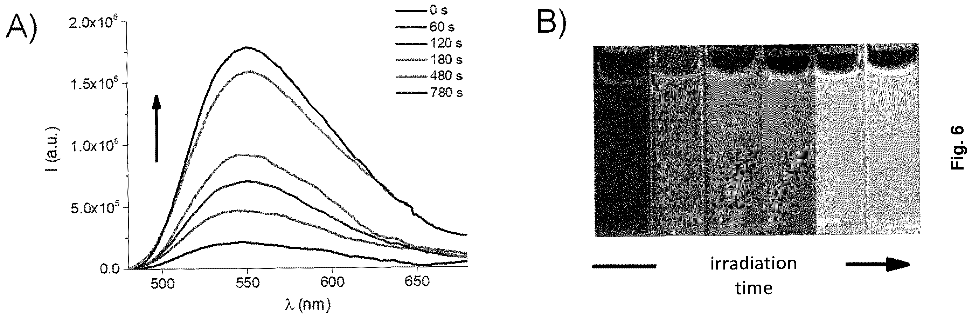

Figure 6 shows the demonstration of what has just been illustrated. The weak emission of conjugate 2 in the green region is significantly increased following irradiation as a result of the formation of the fluorescent reporter (Figure 6A). The fluorescence emission is so intense (quantum efficiency = 0.13) that the release process can also be followed by the naked eye (Figure 6B) which demonstrates that it is possible to monitor the NO release from the reporter emission.

Derivatives 1 and 2 are less soluble than HA in aqueous solution and due to their lipophilicity characteristics they can interact with lipid carriers commonly used for the cutaneous delivery of liposoluble molecules. HA derivatives with NO photodonor can be used in cosmetic and pharmaceutical formulations for topical applications after combination or solubilization in pharmaceutically or cosmetically acceptable semi-solid (creams, gels and ointments) and liquid (lotions, solutions, sprays) vehicles. The components may include lipophilic (fatty acids, fatty alcohols, cholesterol, squalene, triglycerides) or hydrophilic (glycerin, propylene glycol, butylene glycol, panthenol, sorbitol, urea, glycolic acid, lactic acid, pyrrolidone-5-carboxylate) moisturizing ingredients.

As an example, derivative 2 was formulated in a microemulsion of about 40 nm in diameter. The procedure involves the use of Labrafac^{®} Lipophile WL1349 and Kolliphor^{®} HS 15 and water to which HA derivative is added under gentle stirring.

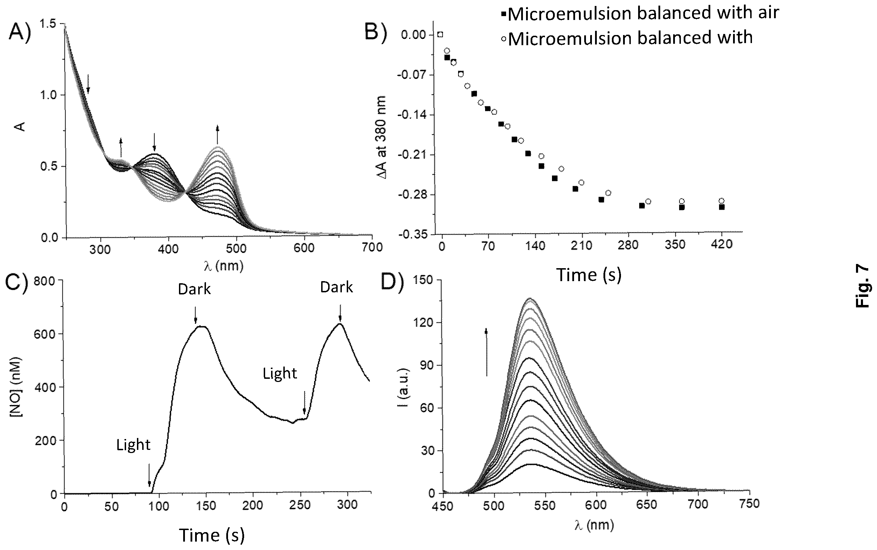

The photochemical properties of the conjugates are perfectly maintained even after their formulation in microemulsions. Figure 7A clearly shows that the changes in the absorption spectra of the microemulsions of 2 upon irradiation with visible light are identical to those observed in the absence of microemulsions (see Figure 3B for comparison). Experiments conducted under anaerobic conditions also demonstrate that the rate of photolysis is independent of the presence of oxygen (Figure 7B). This represents a significant added value, in light of the fact that many light-activated reactions are significantly slowed down by the presence of oxygen, suggesting that the different local oxygen concentration in the skin layers has no effect on the release of NO. Also in this case, the photorelease of NO was unequivocally monitored directly by an amperometric sensor, highlighting that the process is exclusively regulated by the presence of light while it stops instantly in dark conditions (Figure 7C). Finally, similarly to what was found in the absence of microemulsions, the NO photorelease process is accompanied by a drastic increase in fluorescence intensity due to the formation of the fluorescent reporter (Figure 7D).

Similar experiments carried out on the microemulsion containing conjugate 1 have also demonstrated for this compound a maintenance of the NO release properties not influenced by the presence of the microemulsion components or by the oxygen concentration.

The preservation of the photochemical properties of the conjugates of the invention, both in nature and in efficiency, is a non-trivial aspect since the interaction of a photoactivatable system with new components (those of the microemulsion in the present case) can modify both of them even significantly.

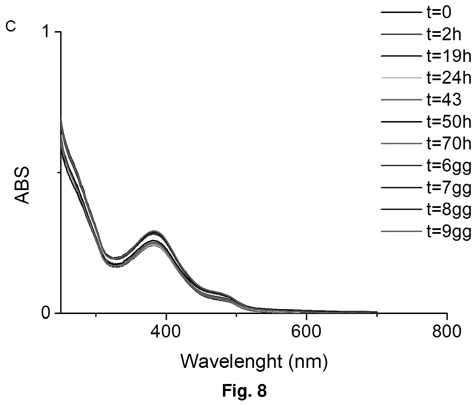

The microemulsions containing conjugates 1 or 2 are stable in the dark. As an example, Figure 8 shows the absorption spectrum of the microemulsion containing conjugate 2 after 9 days.

The ability of the microemulsion to accumulate the conjugate in the deep skin and simultaneously release NO under the action of visible light was demonstrated using the microemulsion containing derivative 2 capable of becoming fluorescent after the photorelease of NO. The experiment was performed on porcine ear skin, which has a structure similar to that of humans and is the most widespread membrane model in skin permeation studies.

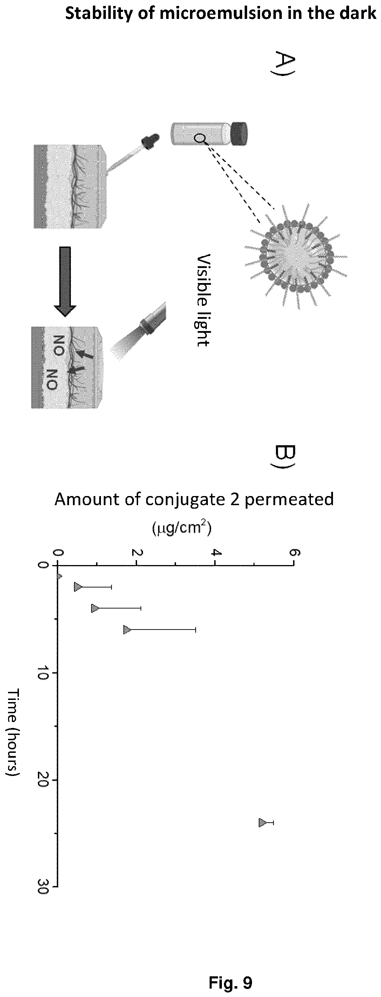

In the transport experiment performed under ambient light shown in Figure 9, it was observed that about 5 µg/cm² of derivative 2 permeated the porcine skin after 24 hours (stratum corneum and epidermis).

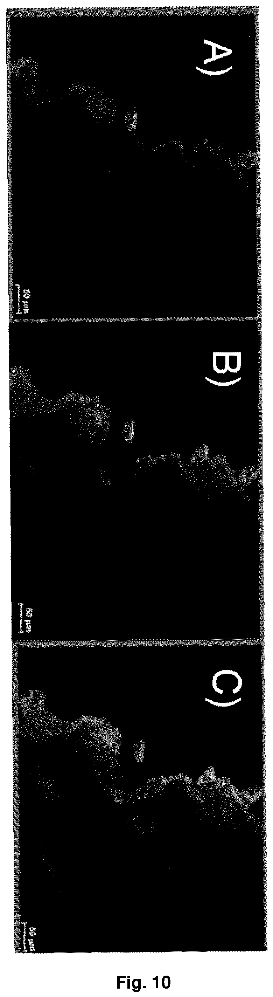

The microemulsion containing derivative 2 allows NO to be released into the skin at concentrations controlled by the duration of exposure to light. As shown in Figure 10, the vertical section of porcine skin on which the microemulsion containing derivative 2 is applied for 4 hours in the dark, shows a low green fluorescence. After progressive photoactivation by blue light, the same section shows an increasingly intense green fluorescence intensity attributable to the increasing amount of the fluorescent reporter, that is to the progressive release of NO determined by the light.

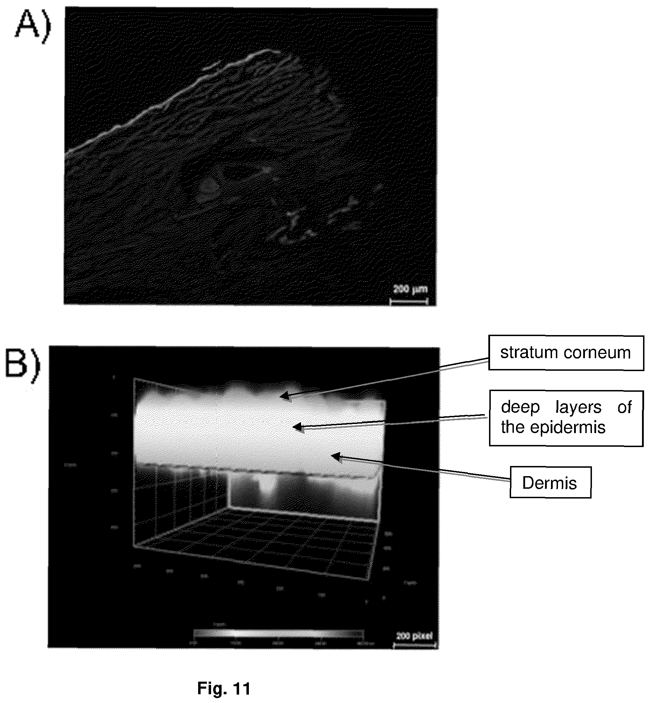

The microemulsion formulation allows conjugate 2 to release NO in the deep layers of the skin and not only on the surface, allowing to exploit the deep regenerating and repairing effect of both HA and NO. As shown in Figure 11, the vertical section of porcine skin on which the microemulsion containing conjugate 2 is applied for 24 hours and exposed to ambient light, shows a high green fluorescence due to the formation of the fluorescent reporter following the release of NO (Figure 11A). Reporter formation is abundant not only in the stratum corneum (red intensity level) but also evident in the deeper layers of the epidermis (yellow intensity level) and in the dermis (green intensity level) at a depth from the surface > 300 µm.

Activation of the conjugates by light allows the prolonged release of NO in the skin until the NO reserve is exhausted, allowing the generation of a NO gradient in the tissue.

The association between HA and a molecule capable of releasing NO determines surprising additive/synergistic effects for cosmetic and therapeutic applications such as:
- Skin aging and wrinkles. HA is used in dermal fillers to increase volume and reduce wrinkles while NO can improve blood circulation, promote collagen production and act as an antioxidant.
- Skin hydration thanks to the ability of medium molecular weight HA to retain water and the vasodilatory effect of NO
- Repairing of chronic wounds, diabetic ulcers, pressure ulcers and burns. HA allows to generate a humid environment, support tissue repair and reduce inflammation while NO promotes vasodilation stimulating the repair process and has antimicrobial properties in infected wounds.
- Skin inflammation such as in the case of eczema, psoriasis, rosacea dermatitis and skin sensitivity thanks to the hydrating properties of immune system modulator and vasodilator.
- Acne and acne scars thanks to the antimicrobial properties of NO and the improvement of skin texture by HA.
- Skin regeneration thanks to the improvement of skin texture, tone and elasticity in cosmetic treatments such as mesotherapy or micro-needling
   as well as in all applications that require the activation of skin and/or dermal repair processes, such as superficial lesions and defects, aging and photoaging, biorevitalization of dermal tissues. In these conditions, the known effects attributable to NO, such as:
   • increased collagen synthesis by keratinocytes;
   • increased microcirculation;
   • facilitation of the proliferation of keratinocytes and fibroblasts;
   • induction of the synthesis of TGF-b1 (Transforming Growth Factor-beta 1) and IL, with consequent activation of skin repair processes;
   • stimulation of chemotactic release factors (such as VEGF - Vascular Endothelial Growth Factor);
   are surprisingly optimized by the association with hyaluronic acid or its derivatives.

The presence of hyaluronic acid or its derivative or salt:
• allows to synergistically exploit the healing, emollient, humectant, repairing and filling properties of hyaluronic acid and/or its derivatives.

Given the modulability of the rheological characteristics of HA as such and its derivatives, very different cutaneous application methods and products adaptable to the application site for the intended use can be obtained. Possible products are an injectable gel for intradermal administration, hydrogels, creams, lotions, advanced medications or thin films for topical applications.

The conjugation between HA and/or its derivatives and the NO source by means of a chemical bond ensures the stability of the product and guarantees that the release of NO occurs where HA is located in a continuous manner.

HA conjugates activated by ambient light, unlike the conjugates that release NO spontaneously, highlight a series of innovative characteristics.
- Release of NO exclusively activated by visible light.
- Stability in physiological conditions and in the absence of light.
- Solubility/lipophilicity characteristics that allow their formulation with lipid components commonly used for the cutaneous delivery of fat-soluble and water-soluble molecules.
- Preservation of the photochemical properties of the conjugates even after their formulation in a product applicable to the skin.
- Delivery into the skin layers by formulation in microemulsions and release of NO activated by visible light in the deep layers.

Considering that:
i) the generation of NO in the skin by chemical means is achieved extemporaneously and only on the external surface and that its consequent deep biological action is highly unlikely due to its short half-life;
ii) the conjugates of HA with spontaneous NO donors are not activated by a specific mechanism that stimulates the release making not only their conservation in a commercial product extremely critical but also the amount of NO released uncontrolled;
   the advantages of the present invention consist in the conjugation of HA with biocompatible NO photogenerators to obtain conjugates capable of:
   i) activating the release of NO directly in the epidermis and not on the surface by exposure to ambient light only after the skin application;
   ii) modulating the release of NO by application of a visible light source;
   iii) allowing the prolonged release of NO in the skin until the reservoir is exhausted, allowing the generation of a NO gradient in the tissue;
   iv) allowing their formulation in lipid components commonly used for the cutaneous delivery of fat-soluble and water-soluble molecules;
   v) obtaining a product stable at room temperature.

The release of NO from the derivatives of the present invention extends well beyond the 500 min reported by the prior art (demonstrated up to 24 hours in porcine skin). The control of the release of NO by means of an external stimulus such as ambient light directly in the skin allows to prolong the positive biological effects of NO throughout the skin thickness. The activation of the release process exclusively by means of light stimuli allows the conservation of the conjugates in the presence of solvents typically used in dermatological preparations, unlike spontaneous donors.

In particular, unlike all the compounds described in WO2009003624 that release NO in a totally spontaneous and uncontrolled manner [15,16, 17], the compounds according to the present invention are advantageously able to stimulate/induce/trigger/favor a controlled/guided/measured NO release both in terms of the quantity of NO generated and in terms of release speed. This release can be carried out thanks to light inputs that, by investing the "hyaluronic acid-NO photodonor conjugates" with electromagnetic radiation at specific wavelengths in the visible spectrum, are able to act as a trigger for the development of NO. Compared to what is evident from the prior art wherein the release of NO can neither be controlled nor voluntarily interrupted, the compounds of the present invention have the surprising advantage of behaving as ON/OFF switches, which can release NO only on command, and therefore in a manner that is anything but spontaneous. The release of NO is in fact an immediate and concomitant response to a received radiative stimulus (external and non-invasive) that stops at the exact moment wherein the light source is turned off. This technical effect therefore allows for accurate and controlled control over the development of NO both in time and space, i.e. as a function of the duration of the light stimulus and the irradiated area. It is thus possible to have total and advantageous control over the NO dosage regimen and the body districts to be subjected to the treatment.

Advantageously, the compounds according to the present invention are the result of a sought-after combination between a NO photodonor with a polymer such as hyaluronic acid. This chemical conjugation cannot be considered as a foregone conclusion and inferable from the known art since unforeseen biomolecular processes could unexpectedly degrade/alter/modify the NO photodonor and/or the polymeric scaffold, consequently leading either to a variation in the release of NO or, in worse cases, even to a total inhibition of the functioning. A simple routine experimentation performed by an expert in the field based on the prior art would therefore not be sufficient to derive the technical teaching of the present invention.

Furthermore, WO2009003624 does not describe and does not suggest changes in permeability of the hyaluronic acid chains once conjugated with NO photodonors. The permeability towards the skin (in particular in the deeper layers of the skin) of the compounds according to the invention cannot therefore be deduced from the prior art, given the lack of scientific support in this regard and given the structural diversity of the compounds. Such permeability has instead been advantageously demonstrated in the present document. The use of low molecular weight HA allows the derivatives of the invention to penetrate deeply through the skin and perform their NO releasing activity more deeply and for a longer time.

The topical formulation of the invention can be used as a cosmetic product and as a pharmaceutical product for all conditions that benefit from the deep action of NO.

In particular, increasing vascularization, collagen production and skin hydration in the cosmetic field, limiting inflammation and the proliferation of skin bacteria such as those responsible for acne (C. *acnes*), increasing vascularization, promoting the repair of chronic wounds and limiting their microbial colonization (*S. epidermidis* and *P. aeruginosa*) in the pharmaceutical field.

The conjugates of the invention can be formulated in emulsions such as fluid lotions, foams, gels, milks and creams suitable for application on the skin. Other possible formulations are: preparations for intradermal administration for the treatment of wrinkles, scars and skin defects; hydrogels, medications or films for topical application with healing, biorevitalizing and regenerating effect. Such formulations can be prepared according to standard procedures and are suitable for application on the skin of a mammal, including humans, including children, newborns and the elderly.

The formulations can also contain one or more: antibiotic agents, antimicrobial agents, antibacterial agents, antifungal agents, anti-inflammatory agents; preservatives and antioxidants. The following can be added: pH regulating agents in the quantities necessary to obtain the desired pH, generally a physiological pH between 5 and 7, preferably between 6 and 7, where lactic acid is preferred; viscosity modifiers, such as xanthan gum.

The expert in the pharmaceutical and/or cosmetic field is able to prepare the cosmetic and pharmaceutical formulations based on his experience and by reading the technical information provided in the following description.

The following examples are provided to illustrate the invention and are not to be considered limiting of the corresponding scope.

### EXAMPLES

### Synthesis of conjugate 1

The synthetic processes involved in the preparation of conjugate 1 are reported below in Scheme 1.

Scheme 1. Reagents and conditions: a) EDC.HCl, EDA.2HCl, MES buffer, rt; b) NOPD_1, DMF, PBS (3 mM, pH = 6.5), rt.
0.3<x<0.7
0.3<y<0.7
n=dependent on molecular weight

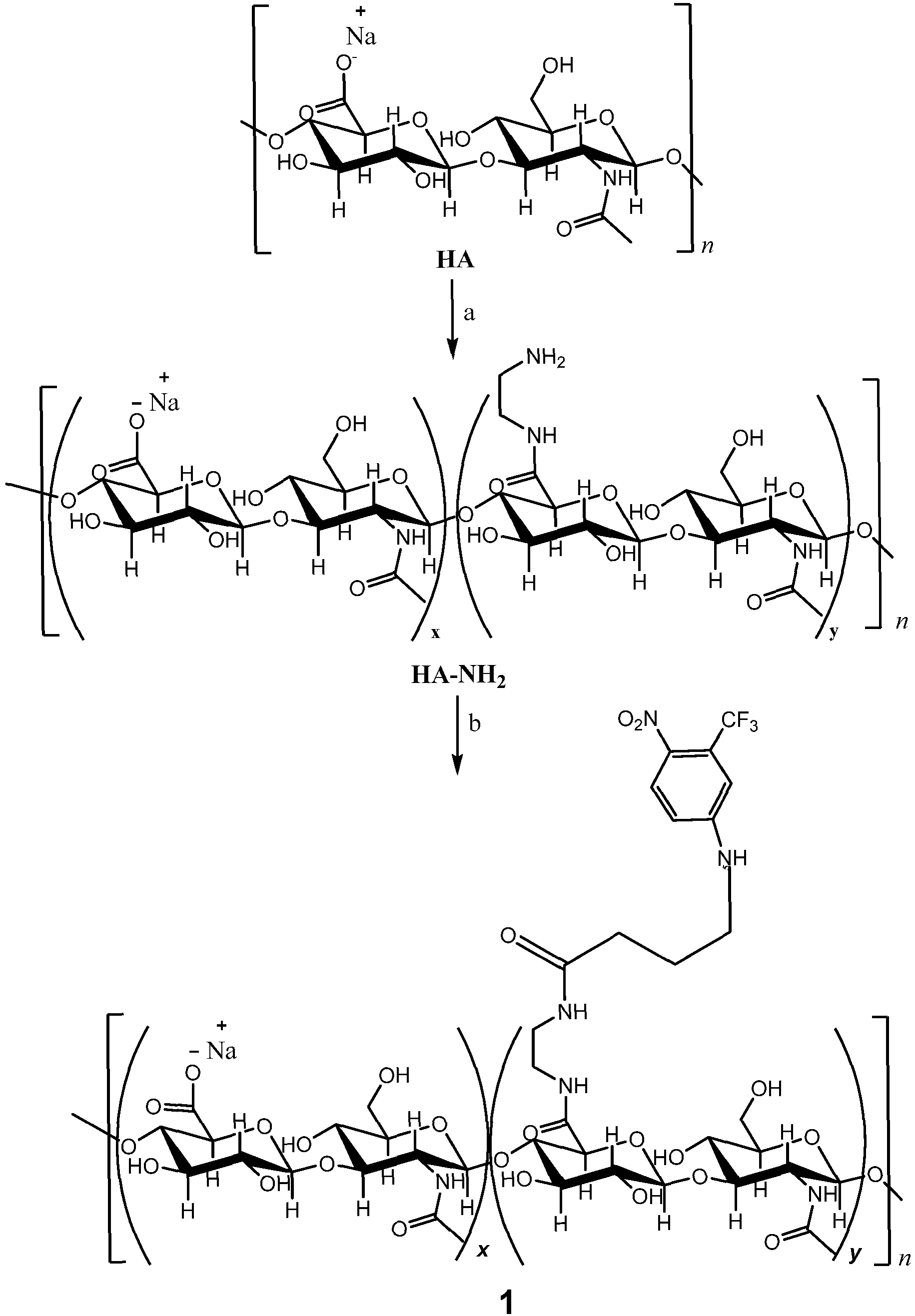

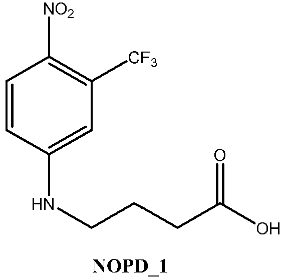

NOPD_1, N-Nitroso-4-((4-nitro-3-trifluoromethylphenyl)amino)butanoic acid was prepared according to the work [12].

**Synthesis of HA-NH₂.** HA-NH₂ was prepared as described by Thierry et al [13], but using 1/0.6 as the ratio of HA/EDA.2HCl equivalents. Specifically, HA (0.374 mmol, 150 mg) and EDC.HCl (0.486 mmol, 93 mg) were dissolved in 10 mL of MES buffer (0.1 M, pH = 5.0). EDA.2HCl (0.224 mmol, 30 mg) was added and the resulting solution was kept overnight at room temperature. The reaction mixture was purified by dialysis against distilled water using a Spectra/Por^{®} regenerated cellulose membrane (Spectrum, Breda, The Netherlands, MWCO = 3.5 KDa) and HA-NH₂ derivative obtained by freeze-drying (140 mg, 92%). As reported by Thierry et al. [13], ¹H NMR spectrum in D₂O confirmed the presence of ethylenediamine groups. The signal at δ = 3.05 ppm attributable to the ethylene protons of the ethylenediamine moieties and the singlet resonance of the methyl acetamide protons at δ = 1.89 ppm, were used to calculate the degree of functionalization of HA-NH₂ (0.3).

### Conjugation of HA-NH₂ with NOPD_1 (conjugate 1).

A solution of NOPD_1 (0.182 mmol, 53 mg) in DMF (2 mL) was added to a solution of HA-NH₂ (0.07 mmol, 30 mg) in phosphate buffer (2 mL, 3 mM, pH 6.5). The resulting mixture was stirred at room temperature for 24 h. Conjugate 1 was purified by dialysis, first against acetone/50% distilled water for 6 h to remove excess NOPD_1, then against distilled water for 24 h, using a Spectra/Por^{®} regenerated cellulose membrane (Spectro, Breda, The Netherlands, MWCO = 3.5 kDa). The sample was finally freeze-dried to obtain conjugate 1 (16 %) in solid form.

### Synthesis of conjugate 2

The synthetic processes involved in the preparation of conjugate 2 are reported in Scheme 2.

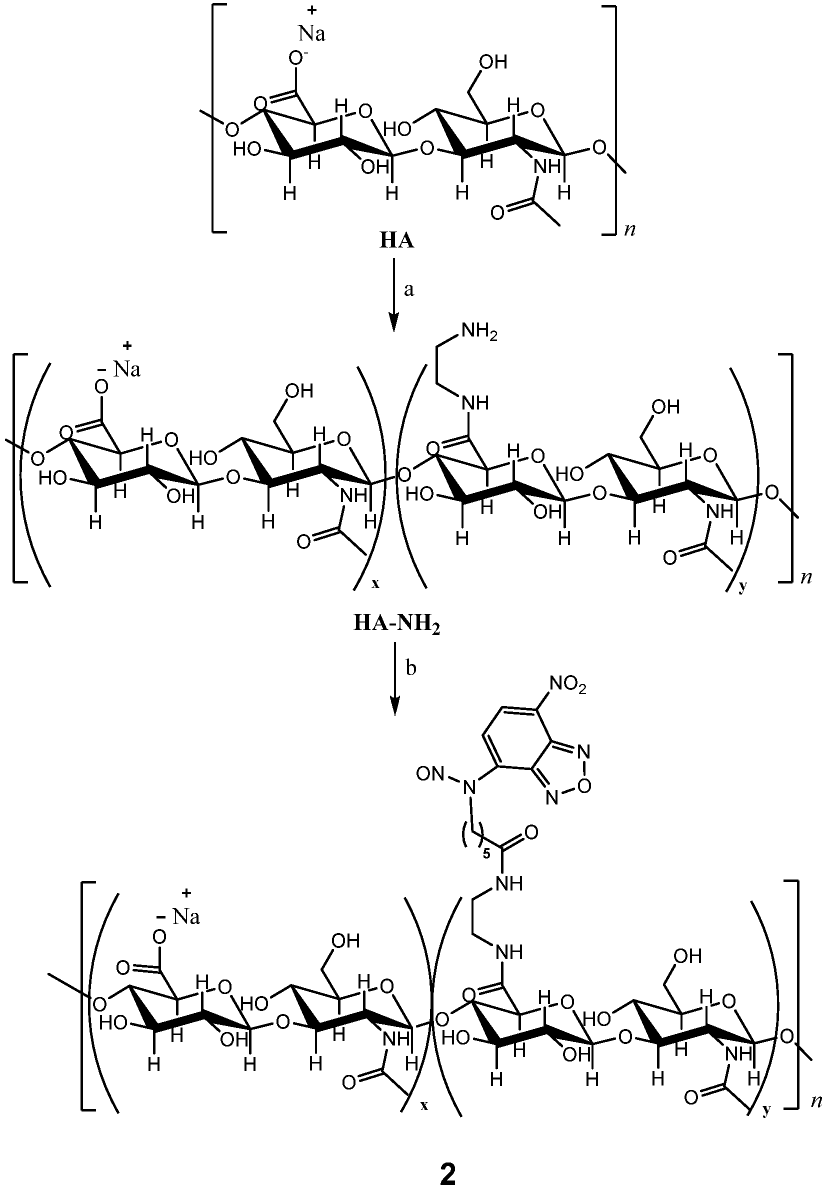

Scheme 2. Reagents and conditions: a) EDC.HCl, EDA.2HCl, MES buffer, rt; b) NBF-NO-NHS, DMF, PBS (3 mM, pH = 6.5), rt.
0.3<x<0.7
0.3<y<0.7
n=dependent on molecular weight 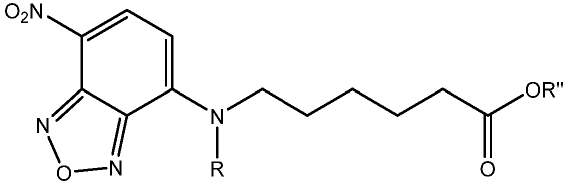
   NBF: R, R" = -H;
   NBF-NO: R = -NO, R" = H; 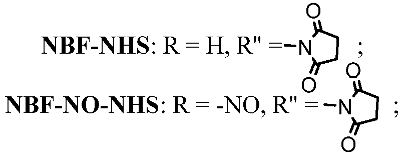
NBF = 6-((7-nitrobenzo[c][1,2,5] oxadiazol-4-yl) amino) hexanoic acid
NBF-NO = 6-((7-nitrobenzo[c][1,2,5] oxadiazol-4-yl) (nitroso)-amino) hexanoic acid
NBF-NHS = 2,5-dioxopyrrolidin-1-yl 6-((7-nitrobenzo[c][1,2,5] oxadiazol-4-yl) amino) hexanoate (NBF-NHS)
NBF-NHS-NO = di 2,5-dioxopyrrolidin-1-yl 6-((7-nitrobenzo[c][1,2,5] oxadiazol-4-yl) (nitroso) amino)hexanoate

### Synthesis of 6-((7-nitrobenzo[c][1,2,5] oxadiazol-4-yl) amino) hexanoic acid (NBF). NBF was prepared as reported in [14]

**Synthesis of 6-((7-nitrobenzo[c][1,2,5] oxadiazol-4-yl) (nitroso)-amino) hexanoic acid (NBF-NO).** NBF-NO was synthesized following our previous procedures [10-11]. Specifically, NBF (0.41 mmol, 120 mg) was dissolved in a THF/CH₃COOH mixture (1/1, 20 mL). After cooling to 0°C with an ice bath, sodium nitrite (1.64 mmol, 114 mg) was added. The reaction mixture was stirred at 0°C for 30 min and then kept overnight at room temperature. The resulting mixture was diluted with DCM and washed with water (3 × 20 mL), dried over Na₂SO4 and concentrated to dryness. The residue was purified by flash chromatography, using petroleum ether/EtOAc/CH₃COOH (60/40/0.5, v/v/v) as eluent. The target compound NBF-NO was obtained as a yellow solid (80 mg, 61%). ¹H NMR (600 MHz, CD₃OD) δ = 8.69 (d, J = 8.2 Hz, ¹H), 7.80 (d, J = 8.2 Hz, ¹H), 4.34-4.31 (m, ²H), 2.25 (t, J = 7.3 Hz, ²H), 1.62-1.54 (m, ⁴H), 1.36-1.29 (m, ²H). ¹³C NMR (150 MHz, CD₃OD) δ = 177.4, 146.7, 146.0, 138.3, 133.4, 116.5, 44.7, 34.6, 27.3, 25.4. ESI-MS [M-H]-: m/z 322.2.

### Synthesis of 2,5-dioxopyrrolidin-1-yl 6-((7-nitrobenzo[c][1,2,5] oxadiazol-4-yl) amino) hexanoate (NBF-NHS). NBF-NHS was synthesized as reported in [14].

**Synthesis of 2,5-dioxopyrrolidin-1-yl 6-((7-nitrobenzo[c][1,2,5] oxadiazol-4-yl) (nitroso) amino) hexanoate (NBF-NHS-NO).** NBF-NHS-NO was prepared according to the previous synthetic strategy [14] but with minor modifications in the purification process. To a solution of NBF-NHS (0.256 mmol, 100 mg) in a THF/CH₃COOH mixture (1/1, 10 mL) cooled to 0°C with an ice bath, sodium nitrite (1.024 mmol, 71 mg) was added. The reaction mixture was stirred for 12 h at room temperature and then diluted with 30 mL of DCM. The resulting sample was partitioned with water twice with 20 mL and the organic layer was concentrated. Purification of the residue by flash chromatography, using petroleum ether/EtOAc (60/40, v/v) as eluent, gave NBF-NHS-NO as a yellow solid (76 mg, 71%). ¹H NMR (600 MHz, CDCl₃) δ = 8.61 (d, J = 8.1 Hz, ¹H), 7.82 (d, J = 8.2 Hz, ¹H), 4.46 - 4.35 (m, 2H), 2.84 - 2.81 (m, ⁴H), 2.62 - 2.51 (m, ²H), 1.89 - 1.83 (m, ²H), 1.66 - 1.59 (m, ⁴H). ¹³C NMR (150 MHz, CDCl₃) δ = 169.5, 169.3, 144.7, 144.0, 137.3, 131.9, 114.6, 60.6, 44.1, 30.7, 26.5, 25.8, 24.0. ESI-MS [M-H]-: m/z 419.5.

**Conjugation of HA-NH₂ with NBF (HA conjugated with fluorescent reporter) and with NBF-NO (conjugate 2).** A solution of NBF-NHS (0.182 mmol, 71 mg) or NBF-NO-NHS (0.182 mmol, 77 mg) in DMF (2 mL) was added to a solution of HA-NH₂ (0.07 mmol, 30 mg) (prepared according to the procedure already reported for conjugate 1) in phosphate buffer (2 mL, 3 mM, pH 6.5). The resulting mixture was stirred at room temperature for 24 h. Conjugate 2 was purified by dialysis, first against 50% acetone/distilled water for 6 h to remove excess NBF-NHS or NBF-NO-NHS, then against distilled water for 24 h, using a Spectra/Por^{®} regenerated cellulose membrane (Spectro, Breda, The Netherlands, MWCO = 3.5 kDa). The sample was finally freeze-dried to obtain HA-NBF (18 mg, 60%) or conjugate 2 (20 mg, 67%) in solid form.

### FORMULATION OF THE MICROEMULSION (ME)

500 µL of Kolliphor^{®} HS 15 and 250 µL of Labrafac^{®} Lipophile WL1349 are placed under magnetic stirring for 10 minutes. Then the aqueous phase (4.25 mL) is added, always under magnetic stirring. The ME is left under stirring for 30 minutes and then at room temperature for 2 hours. After this time, 0.5 mg of conjugate 1 or 2 are added to 2 mL of ME under magnetic stirring at 900 RPM and then left to stir for 3 hours.

For permeation studies, MEs were prepared containing, in addition to conjugate 2, indocyanine green (IG) as a fluorescent probe: 1 mg of IG was solubilized in 500 µL of Kolliphor^{®} HS 15 and 250 µL of Labrafac^{®} Lipophile WL1349 under magnetic stirring for 10 minutes. Subsequently, the aqueous phase (4.25 mL) was added under electromagnetic stirring. The ME was left under stirring for 30 minutes and then left at room temperature for 2 hours. After this time, 0.5 mg of conjugate 2 was added to 2 mL of ME under magnetic stirring at 900 RPM and then left to stir for 3 hours.

The hydrodynamic diameter (DH), the polydispersity index (Pdl) and the zeta potential (ζ) of the MEs were evaluated on the Zetasizer Nano ZS.

The stability of the MEs loaded with conjugates 1 and 2 was monitored by spectroscopic and colloidal analyses in three different conditions: in the light and in the dark at room temperature and in the dark at 4°C. The photodegradation studies were carried out by inserting the microemulsion containing conjugate 2 (conc. 0.25 mg/mL) in a 1 mm cuvette and monitoring the absorption spectrum by means of a Shimadzu UV-1800 spectrophotometer.

Implementation variants of the non-limiting examples described are possible, without however departing from the scope of protection of the present invention, including all the embodiments equivalent for a technician in the field, to the content of the claims.

From the description reported above, the technician in the field is able to achieve the object of the invention without having to introduce further details.

### References

[1] Volpi, N. et al., Role, Metabolism, chemical modifications and applications of hyaluronan. Curr. Med. Chem. 2009, 16, 1718-1745.
[2] Juncan AM, et al., Advantages of Hyaluronic Acid and Its Combination with Other Bioactive Ingredients in Cosmeceuticals. Molecules. 2021 Jul 22;26(15):4429.
[3] Essendoubi, M. et al., Human skin penetration of hyaluronic acid o different molecular weights as probed by Raman spectroscopy. Ski. Res. Technol. 2016, 22, 55-62.
[4] Nitric Oxide: Biology and Pathobiology, 3rd ed., Ed. Ignarro, L. J.; Freeman B.A.; Elsevier Inc., 2017.
[5] Adler BL. et al. Nitric oxide therapy for dermatologic disease. Future Sci OA. 2015 Aug 1;1(1):FSO37.
[6] E. B. Caruso, S. Petralia, S. Conoci, S. Giuffrida, S. Sortino, J. Am. Chem. Soc., 2007, 129, 480;
[7] S. Conoci, S. Petralia and S. Sortino, EP 2051935A1/US 20090191284, 2006
[8] M. Malanga, M. Seggio, V. Kirejev, A. Fraix, I. Di Bari, E. Fenyvesi, M. B. Ericson and S. Sortino, A phototherapeutic fluorescent b-cyclodextrin branched polymer derivering nitric oxide. Biomater. Sci. 2019, 7, 2272-2276.
[9] Chegaev, K.; Fraix, A.; Gazzano, E.; Abd-Ellatef, G. E. F.; Blangetti, M.; Rolando, B.; Conoci, S.; Riganti, C.; Fruttero, R.; Gasco, A.; Sortino, S. Light-regulated NO release as a novel strategy to overcome doxorubicin multidrug resistance. ACS Med. Chem. Lett. 2017, 8, 361-365.
[10] C. Parisi, M. Seggio, A. Fraix, S. Sortino, A high-performing metal-free photoactivatable NO donor with a green fluorescent reporter, ChemPhotoChem 4 (2020) 742-748.
[11] A. Fraix, C. Parisi, M. Seggio and S. Sortino Nitric Oxide Photoreleasers with Fluorescent Reporting Chem. Eur. J. 2021, 27, 12714-1272.
[12] C. Parisi, M. Failla, A. Fraix, L. Menilli, F. Moret, E. Reddi, F. Spyrakis, B. Rolando, L. Lazzarato, R. Fruttero, A. Gasco and S. Sortino A Generator of Peroxynitrite Activatable with Red Light, Chem. Sci., 2021, 12, 4740
[13] Thierry, B.; Kujawa, P.; Tkaczyk, C.; Winnik, F.M.; Bilodeau, L.; Tabrizian, M. Delivery platform for hydrophobic drugs: prodrug approach combined with self-assembled multilayers. J. Am. Chem. Soc., 2005, 127: 1626-1627
[14] Greco, G.; D'Antona, N.; Gambera, G.; Nicolosi, G. Glycerophosphoinositols: Total Synthesis of the First Fluorescent Probe Derivative. SYNLETT, 2014, 25: 2111-2114.
[15] ACS Appl Bio Mater. 2022 May 16; 5(5): 2285-2295.
[16] Chem.Rev. 2002, 102, 1091-113.
[17] J. Chem. Soc., Perkin Trans. 2, 1997, 351.

## Claims

1. A conjugate of hyaluronic acid (HA), its salts, which is covalently linked with an organic nitric oxide (NO) photodonor compound capable of releasing NO by stimulation with electromagnetic radiation in the range between 300 and 800 nm, preferably in the range 400-700 nm.

2. The conjugate according to the preceding claim which has the general formula (I) 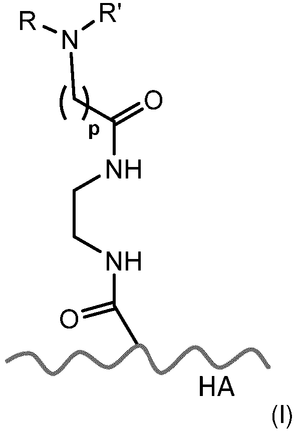 wherein:
HA = hyaluronic acid backbone
p = positive integer between 3 and 16
R = H, NO
R' = nitrophenyl substituted with, in ortho position with respect to the nitro group, one or two methyl or trifluoromethyl groups, preferably 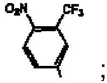 nitrobenzofurazane

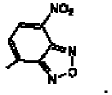

3. The conjugate according to anyone of claims 1-2 wherein R' is selected from

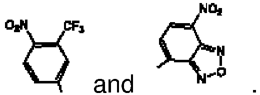

4. The conjugate according to anyone of claims 1-3 wherein the hyaluronic acid derivative is selected from: HA esters with alcohols of the aliphatic, arylaliphatic, cycloaliphatic, aromatic, cyclic and heterocyclic series; HA amides with amines of the aliphatic, arylaliphatic, cycloaliphatic, aromatic, cyclic and heterocyclic series; O-sulfated derivatives of HA up to the 4th degree of sulfation; internal esters of HA with an esterification percentage not exceeding 20%, preferably between 0.05 and 10% of esterification, and with the remaining percentage of non-esterified HA which can be salified with organic and/or inorganic bases; HA derived from the deacetylation of N-acetylglucosamine residues present in HA, with a deacetylation percentage preferably between 0.1 and 30%, while all the carboxylic groups of HA can be salified with organic and/or inorganic bases; percarboxylated derivatives of HA obtained by oxidation of the primary hydroxyl of the N-acetylglucosamine fraction with a degree of percarboxylation between 0.1 and 100%, and preferably between 25 and 75%, the carboxylic groups of HA being salificable with organic and/or inorganic bases.

5. The conjugate according to anyone of claims 1-4 wherein the molecular weight of HA varies in the range between 400 and 3×10⁶ Da, preferably between 200,000 and 750,000 Da, and even more preferably between 8000 Da and 15,000 Da.

6. The conjugate according to anyone of claims 1-4 wherein the hyaluronic acid has a molecular weight selected from: hyaluronic acid with a molecular weight >5000 kDa; hyaluronic acid with a molecular weight in the range 1000-3000 kDa; hyaluronic acid with a molecular weight in the range 250-1000 kDa; 10-250 kDa; < 10 kDa; and mixtures thereof.

7. The conjugate according to anyone of claims 1-6 that releases NO at the level of the epidermis and dermis when exposed to electromagnetic radiation in the range between 300 and 800 nm, preferably in the range 400-700 nm.

8. Compositions comprising the conjugate according to anyone of claims 1-7.

9. The compositions according to the preceding claim formulated as gels, hydrogels, films, injectable gels with controlled viscosity for drug delivery, emulsions and microemulsions, creams, gels, ointments, solutions, sprays, lotions, fluid lotions, foams, milks; in particular microemulsions.

10. The compositions according to the preceding claim wherein the substituent R' of the conjugate according to anyone of claims 1-2 is selected from 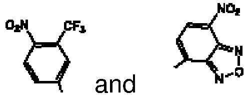 and the formulation is a microemulsion.

11. The compositions according to anyone of claims 8-10 to be applied topically or intradermally.

12. The compositions according to anyone of claims 8-11 further comprising one or more: antibiotic agents, antimicrobial agents, antibacterial agents, antifungal agents, anti-inflammatory agents; preservatives and antioxidants, pH regulators such as lactic acid; viscosity modifiers, such as xanthan gum, cosmetically acceptable components.

13. The conjugate or composition according to anyone of claims 1-11 for use in the medical field.

14. The conjugate or composition according to anyone of claims 1-12 wherein the conjugate or composition is for use in the treatment of conditions that benefit from the release of NO at the skin level in animals and humans, in particular for children, newborns and the elderly, in order to preserve the integrity and functionality of the skin.

15. The conjugate or composition according to anyone of claims 1-12 wherein the conjugate or composition is for use in the treatment of acute and chronic skin and/or dermal lesions, in particular for the repair of chronic wounds, diabetic ulcers, pressure ulcers and burns and to support tissue repair and reduce inflammation by promoting vasodilation and stimulating the repair process.

16. The conjugate or composition according to anyone of claims 1-12 wherein the conjugate or composition is for use in the treatment of infections of the skin, dermis and related lesions, preferably infections by *S. epidermidis*, *C. acnes*, *P. aeruginosa*, skin inflammations such as those caused by eczema, psoriasis, rosacea dermatitis; conditions that benefit from an immunomodulatory and antimicrobial activity in infected wounds.

17. The conjugate or composition for use according to anyone of claims 13-16 wherein the conjugate or composition is administered topically.

18. Use of the conjugate or composition according to anyone of claims 1-12 in the cosmetic field.

19. Use of the conjugate or composition according to the preceding claim to increase the volume of the skin and reduce wrinkles, improve blood circulation, promote collagen production, act as an antioxidant, increase vascularization and skin hydration, limit inflammation.

20. The use of the conjugate or composition according to anyone of claims 18-19 in the preparation of injectable gels for the treatment of wrinkles, scars and skin defects, such as those caused by acne; hydrogels, medications or films for topical application for the improvement of skin texture and with a healing, biorevitalizing and regenerating effect; injectable gels with controlled viscosity for drug delivery.

21. A non-therapeutic method for the skin treatment of a subject, comprising intradermal injection, in particular mesotherapy and micro-needling, of the conjugate or composition according to anyone of claims 1 to 12.
